# EUROPEAN PATENT SPECIFICATION

(11) **EP 1 830 879 B1**
(45) Date of publication and mention of the grant of the patent: **27.10.2010**
(21) Application number: 05853048.6
(22) Date of filing: 02.12.2005
(51) Int. Cl.: A61K 41/00, A61K 49/12, A61K 47/48, A61P 35/00

(54) **MRI GUIDED PHOTODYNAMIC THERAPY FOR CANCER**
MRT-GEFÜHRTE PHOTODYNAMISCHE THERAPIE GEGEN KREBS
THERAPIE PHOTODYNAMIQUE ANTI-CANCEREUSE GUIDEE PAR IRM

(30) Priority: 03.12.2004 US 633255 P
(43) Date of publication of application: 12.09.2007
(73) Proprietor: University of Utah Research Foundation, Salt Lake City, UT 84108 (US)
(72) Inventor: LU, Zheng-Rong, Salt Lake City, Utah 84108 (US); VIADYA, Anagha, Buffalo Grove, IL 60089 (US); KE, Tianyi, Salt Lake City, Utah 84102 (US)
(74) Representative: Campbell, Neil Boyd
(86) International application number: PCT/US2005/044012
(87) International publication number: WO 2006/060797

(56) References cited:
- EP-A- 1 110 963
- US-A- 5 594 136
- US-A- 6 114 321
- US-B1- 6 340 461
- YOUNG STUART W ET AL: "Gadolinium (III) texaphyrin: a tumor selective radiation sensitizer that is detectable by MRI" PROCEEDINGS OF THE NATIONAL ACADEMY OF SCIENCES OF USA, NATIONAL ACADEMY OF SCIENCE, WASHINGTON, DC, vol. 93, 1 January 1996 (1996-01-01), pages 6610-6615, XP002178040 ISSN: 0027-8424
- LU Z -R ET AL: "Poly(L-glutamic acid) Gd(III)-DOTA conjugate with a degradable spacer for magnetic resonance imaging" BIOCONJUGATE CHEMISTRY 200307 US, vol. 14, no. 4, July 2003 (2003-07), pages 715-719, XP002490707 ISSN: 1043-1802
- SHIAH J -G ET AL: "Antitumor activity of N-(2-hydroxypropyl)methacrylamide copolymer- mesochlorin e6 and Adriamycin conjugates in combination treatments" March 2000 (2000-03), CLINICAL CANCER RESEARCH 200003 US, VOL. 6, NR. 3, PAGE(S) 1008 - 1015 , XP002490708 ISSN: 1078-0432 * page 1012, column 2, line 13 - line 15 *

## Description

### CROSS-REFERENCE TO RELATED APPLICATION

This application claims priority to US provisional application number 60/633,255, filed December 3, 2004.

### STATEMENT REGARDING FEDERALLY SPONSORED

### RESEARCH OR DEVELOPMENT

Work described herein was supported in part by a grant from the National Institutes of Health, grant number CA097465. The United States Government may have certain rights in the invention.

### TECHNICAL FIELD

The invention relates to the field of biotechnology and cancer treatment, and more particularly to the use of magnetic resonance imaging-guided photodynamic treatment of tumors and other target tissues and/or sites.

### BACKGROUND OF THE INVENTION

As disclosed in U.S. Patent Application 20020095197, PDT (*i*.*e*., photochemotherapy) is an emerging cancer treatment based on the combined effects of visible light and a photosensitizing agent that is activated by exposure to light of a specific wavelength. Photochemotherapy is well known (Hsi R. A., Rosenthal D. I., Glatstein E., "Photodynamic therapy in the treatment of cancer: current state of the art," Drugs 1999, 57(5):725-734; Moore J. V., West C. M. L., Whitehurst C., "The biology of photodynamic therapy," Phys. Med. Biol. 1997; 42:913-935), and, as currently performed for cancer therapy, a photosensitizing agent is injected into a subject systemically, which results in preferential uptake of the photosensitizing agent in tumor cells. The tumor site is then illuminated with visible light of a particular energy and wavelength that is absorbed by the photosensitizing agent. This illumination activates the photosensitizing agent, for instance, resulting in the generation of cytotoxic excited state oxygen molecules in those cells in which the agent has localized. These molecules are highly reactive with cellular components, and provide a treatment (a decrease in size, number, or mass) of tumor cells. Photodynamic therapy initially garnered clinical interest in the mid-20^{th} century when it was demonstrated that porphyrin compounds accumulated preferentially in tumors, resulting in photosensitization and, due to the fluorescence of these compounds, aided in tumor detection. Dougherty is credited with the creation of modem photodynamic therapy, recognizing the potential of photodynamic therapy for tumor treatment and demonstrating its use in treating metastatic tumors of the skin in the 1970's (Oleinick N. L., Evans H. H., "The photobiology of photodynamic therapy: cellular targets and mechanisms," Radiat. Res. 1998; 150:S146-56).

As disclosed in U.S. Patent 6,825,343, photodynamic therapy ("PDT") generally involves the administration of compounds that are capable of absorbing light, typically in the visible range, (but also in the near ultraviolet), followed by irradiation of locations of the subject for which a modifying or inhibitory effect is desired. PDT was initially developed using hematoporphyrin and related compounds to treat tumors, as it appeared that these compounds would localize in rapidly dividing cells (such as in tumors). The tumor could then be irradiated with light. The light is absorbed by the hematoporphyrin and the tumor destroyed. PDT has since been shown to be useful for treating of atherosclerotic plaques, restenosis, infections in the blood stream, rheumatoid arthritis, psoriasis and in the treatment of ocular conditions not necessarily limited to tumors.

Use of the paramagnetic metal ion lanthanoid, gadolinium, in magnetic resonance imaging ("MRI") is well documented. See, for instance, U.S. Patent Application 20040204344 and references therein. Paramagnetic metal chelates Gd(III)-DTPA, Gd(III)-DOTA, and their derivatives increase the relaxation rate of surrounding water protons and are used as contrast agents for MRI⁽¹⁾.

However, low molecular weight contrast agents cannot effectively discriminate diseased tissue from normal tissues. Macromolecular Gd(III) complexes have been developed by conjugating these Gd(III) chelates to bio-medical polymers, including poly(amino acids)^{(2, 3)}, polysaccharides^{(4, 5)}, dendrimers⁽⁶⁻⁸⁾, and proteins^{(9, 10)}, to improve image contrast enhancement. EP110963, US6114321 and Young et al., Proc. Natl. Acad. Sci., 1996, 13, 6610-6615 disclose the use of a photosensitizer bound to a Gd(III) complex as a way by which improved localisation of MRI treatment to tumor tissue may be achieved. Furthermore, US 5594 136 discloses the incorporation of Gd(III) into a copolymer. These macromolecular agents have demonstrated superior contrast enhancement for blood pool imaging and cancer imaging in animal models. Unfortunately, the clinical application of macromolecular agents is limited by their slow excretion after MRI exams^{(11, 12)} and potential unwanted side-effects of Gd(III) ions released by the metabolism of the agents⁽¹³⁻¹⁵⁾.

Recently, there have been some efforts to facilitate the clearance of macromolecular Gd(III) complexes. For example, lysine has been co-injected with dendrimer-based macromolecular agents to facilitate their renal clearance by blocking the renal tubular reabsorption⁽¹⁶⁾. Zheng-Rong Lu reports one example of such poly(L-glutamic acid) Gd(III)-DOTA conjugates with degradable spacers⁽¹⁷⁾. However, this approach cannot facilitate the clearance of macromolecules of relatively high molecular weights. Innovative design of macromolecular Gd(III) complexes is needed to accelerate the clearance of Gd(III) complexes after the MRI examinations and to reduce the potential side affects of macromolecular contrast agents.

Currently available MRI-guided therapies for cancer, like LITT (Laser-Induced Interstitial Thermotherapy), or RFA (Radio Frequency Ablation), are invasive and have problematic disadvantages such as difficulty in treating non-uniform lesions and safety concerns due to skin bums.

Also, currently used low molecular weight contrast agents have fast clearance, low relaxivity, and lower contrast enhancement, especially in tumors. Thus, high molecular weight contrast agents are now more commonly used to passively target tumors (due to the EPR effect). These higher molecular weight contrast agents show higher relaxivity and consequently better contrast enhancement in tumors. In non-MRI applications, localisation of tumor tissue has been shown to be enhanced by the formation of a polymer conjugate of mesochlorin e6 by Shiah et al, Clinical Cancer Res., 2000, 6, 1008-1015.

### SUMMARY OF THE INVENTION

The present invention involves a new technology for cancer treatment with MRI-guided photodynamic cancer therapy. In one embodiment, this technology includes administration of MRI contrast agent labeled polymer-photosensitizer conjugates, detection and localization of tumor or cancer tissues with contrast-enhanced MRI and illumination of target tissues, such as, tumor or cancer tissues, with a laser. The delivered laser energy will activate the photosensitizer accumulated in the target tissue, resulting in target cell death and treatment. This method, as disclosed herein, is more non-invasive as compared to other image-guided therapies, including image-guided ablation. The present invention includes MRI contrast-agent-labeled polymer-photosensitizer conjugates as defined in claim 7 in a combination of contrast-enhanced MRI with photodynamic therapy.

Herein is disclosed the synthesis of high molecular weight poly-L-glutamic acid (PLGA) - Gadolinium ("Gd") complexes containing a photosensitizer drug (Mesochlorin *e*₆) and contrast agent (DOTA-Gd), which are covalently attached to a polymeric backbone. These complexes show significant contrast enhancement in tumors after a delay as compared to other complexes currently used in the field, and may be used for MRI-guided PDT.

Contrast-enhanced MRI is an effective approach to non-invasive tumor detection. Photodynamic therapy (PDT) is a clinically used therapy in the treatment of diseases, such as cancer. Embodiments of the invention combine contrast-enhanced MRI and PDT to provide MRI-guided photodynamic therapy.

In this invention, the drug delivery system contains an MRI contrast agent and a photosensitizer covalently bonded to a polymer wherein the polymer is poly-L-glutamic acid, the contrast agent is Gd-DOTA, and the photosensitiser is mesochlorine. The delivery system carries the contrast agent and photosensitizer into the target tissue, which tissue is then localized in the subject by contrast-enhanced MRI. A laser beam is directed to the target tissue site. Laser energy activates the photosensitizer, which, in an embodiment, generates highly reactive species that kill or destroy the target cells and tissue.

In one exemplary embodiment, said delivery system is used to treat cancer or tumor cells.

In an exemplary embodiment, the photosensitizer conjugates as defined above are used to detect and treat breast cancer cells. In other exemplary embodiments, the delivery system of the present invention is used to detect and treat breast cancer cells, pancreatic cancer cells, prostate cancer cells, skin cancer cells, and other malignancies able to be treated using the method of the present invention.

In another embodiment, provided is a method of synthesis of novel MRI contrasting agents conjugated to photosensitizers useful in the treatment of target tissues in a subject.

### DESCRIPTION OF THE FIGURES

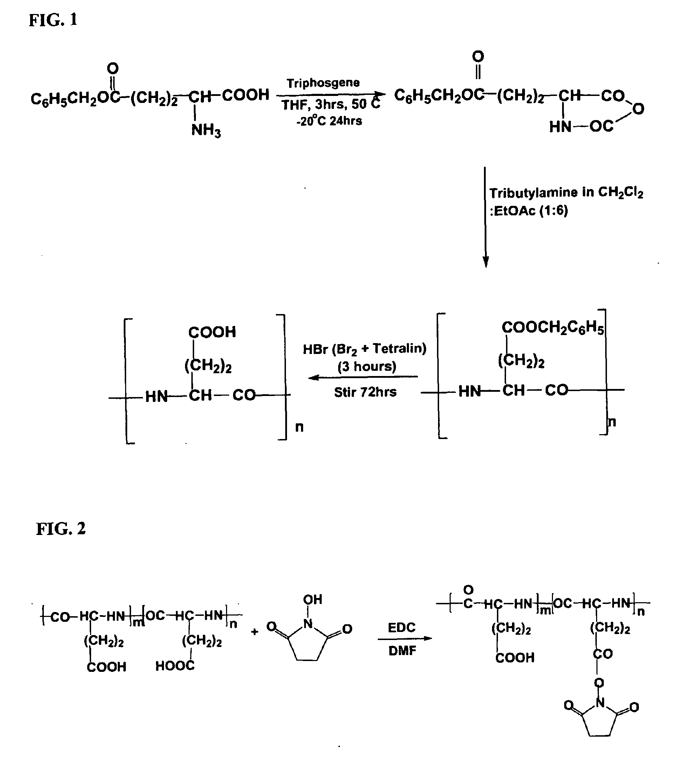

FIG. 1. Synthesis of Poly-L-Glutamic Acid. γ-Benzyl glutamate is reacted with triphosgene in tetrahydrofuran ("THF") for three hours at 50°C, then cooled to -20°C for 24 hours to produce the *N*-carboxy anhydride ("NCA") of γ-benzyl glutamate. This product is then reacted with tributylamine in dichloromethane and ethylacetoacetate ("EtOAc," 1:6, v:v) to produce poly-(benzyl glutamic acid). Poly-(benzyl glutamic acid) is then incubated with hydrogen bromide (Br₂ + tetralin) for three hours and stirred for 72 hours to yield poly-(*L*-glutamic acid).

FIG. 2. Synthesis of Poly-L-Glutamic Acid Active Ester. Poly(L-glutamic acid) ("PLGA," MW 67 kDa, 810 mg) is dissolved in 4 ml of *N,N*-dimethylformamide ("DMF") with 2.166 g of N-hydroxy succinimide ("NHS," MW 115, 3X moles based on glutamic acid monomer). A minimum amount of DMF is used. After complete dissolution of the PLGA (about one hour) and NHS in DMF, 3.61 g of 1-ethyl-3-(3-dimethyl aminopropyl)carbodiimide ("EDC," MW 191, 3X moles) is added. The reaction is stirred for 24 hours and after completion of reaction, DMF is evaporated. The reaction mixture is precipitated in acetone and dried under vacuum and analyzed by nuclear magnetic resonance ("NMR").

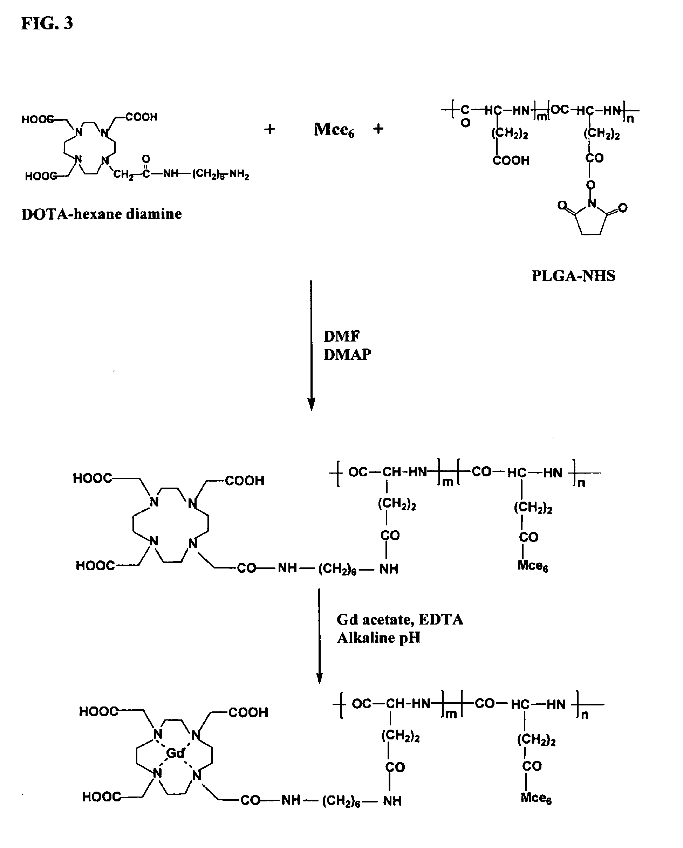

FIG. 3. Synthesis of PLGA-Mce₆-DOTA-Gd Complexes. PLGA-NHS, mesochlorin *e*₆ ((2*S*, 3*S*)-18-carboxy-20-(carboxymethyl)-8, 13-diethyl-3, 7, 12, 17-teframethyl-chlorin-2-propionic acid, "Mce₆") and 1, 4, 7,10-Tetraazacyclododecane-N,N',N",N"'-tetraacetate ("DOTA")-hexane diamine are incubated in dimethylaminopyridine ("DMAP") and DMF for 24 hours. The DMF is evaporated and the conjugate dissolved in water using 0.1 N NaOH and adjusted to pH 11. Mce₆ and other unreacted components are extracted and the product reacted with excess Gd(OAc)₃. After reaction for 24 hours, ethylenediaminetetraacetic acid ("EDTA") is added and the product purified and lyophilized. (*See*, Example I, *infra*.)

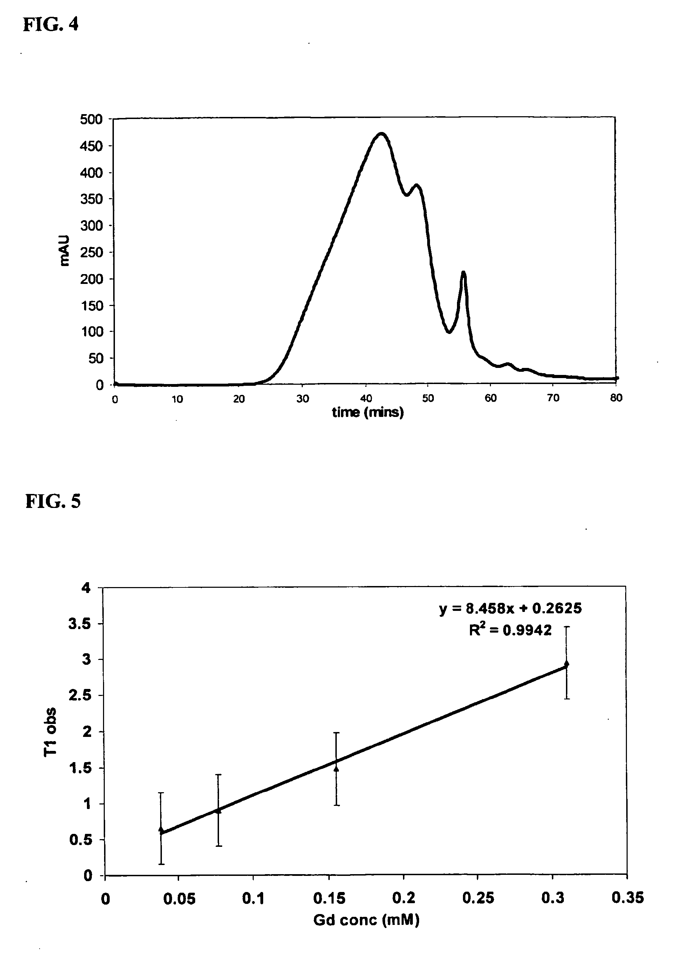

FIG. 4. Determination of Molecular Weight of Polymer-Gd Complexes. Plot of elution profile, milli-absorbane units ("mAU") v. time in minutes, showing elution of polymer-Gd complexes. Sample was applied to a Sepharose 6 size-exclusion gel FPLC column (Pharmacia) at 0.5 ml/min in Tris buffer with 30% acetonitrile.

FIG. 5. Determination of Relaxivity of Polymer-Gd Complexes. Plot of T1 v. [Gd] in mM allows determination of the relaxivity of the polymer-Gd complex. The T1 of Polymer-Gd complexes at various concentrations in aqueous solution were measured using B 1 homogeneity corrected Look-Locker technique on a 1.5T GE NV/CVi scanner with the LX 8.4 operating system at room temperature⁽¹⁸⁾. The coordination of bulky water molecules is crucial for the relaxivity of Gd(III) complexes.

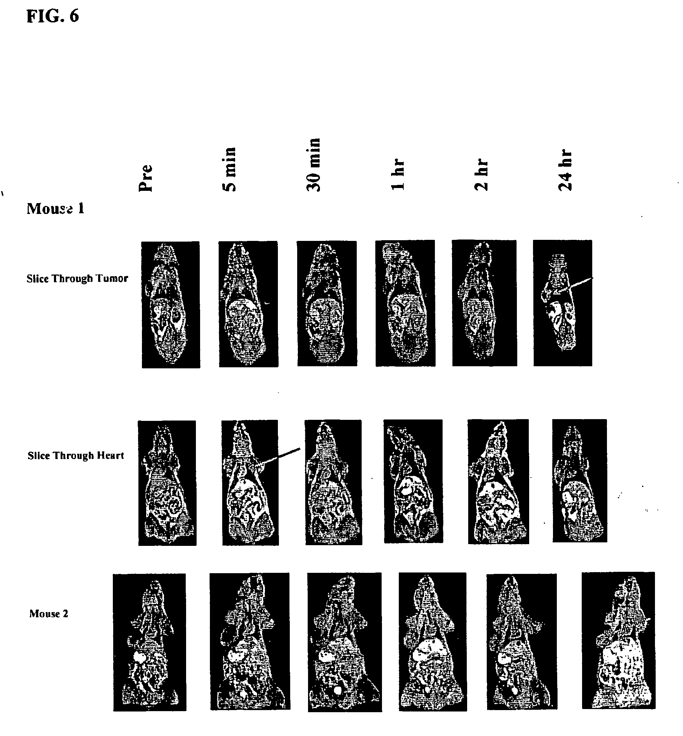

FIG. 6. MRI of tumors (athymic nu/nu mice) at pre-, 5, 30, 60, 120 minutes and 24 hours post-injection. The slices are through the heart and tumor separately, for mouse 1, and through the heart and tumor simultaneously, for mouse 2. In both mice, the 24 hour images show contrast enhancement through the tumor as compared to pre-contrast while there is contrast enhancement in the heart at five minutes post-injection.

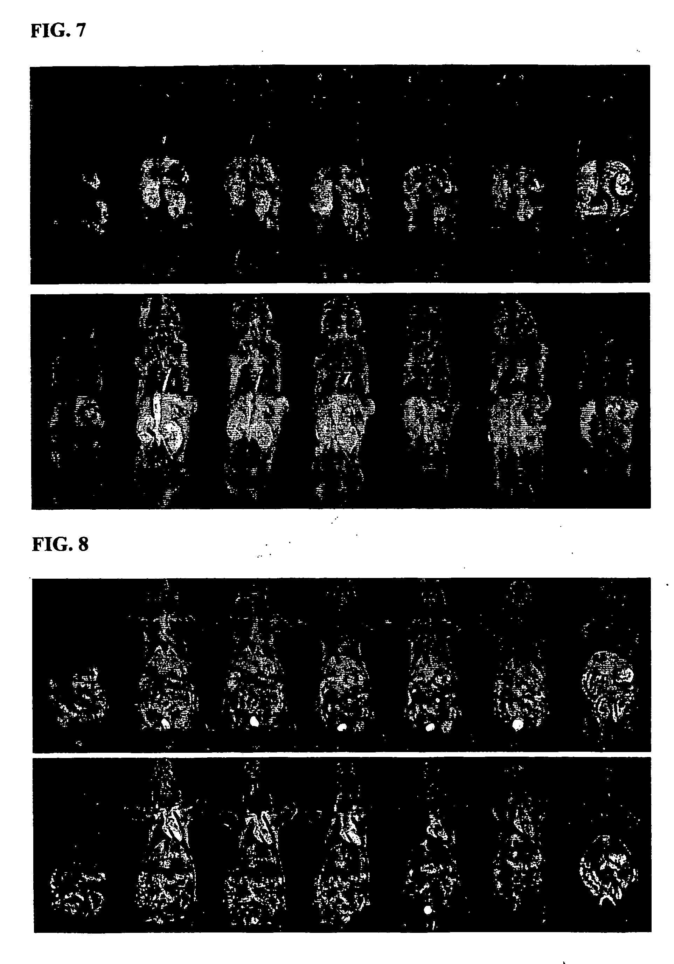

FIG. 7. 2D coronal MR images for animals receiving polymer with Mce6 (Pol1, upper panel) and control (Pol2, lower panel) through tumor cells.

FIG. 8. 2D coronal MR images for animals receiving polymer with Mce6 (Pol1, upper panel) and control (Pol2, lower panel) through heart cells.

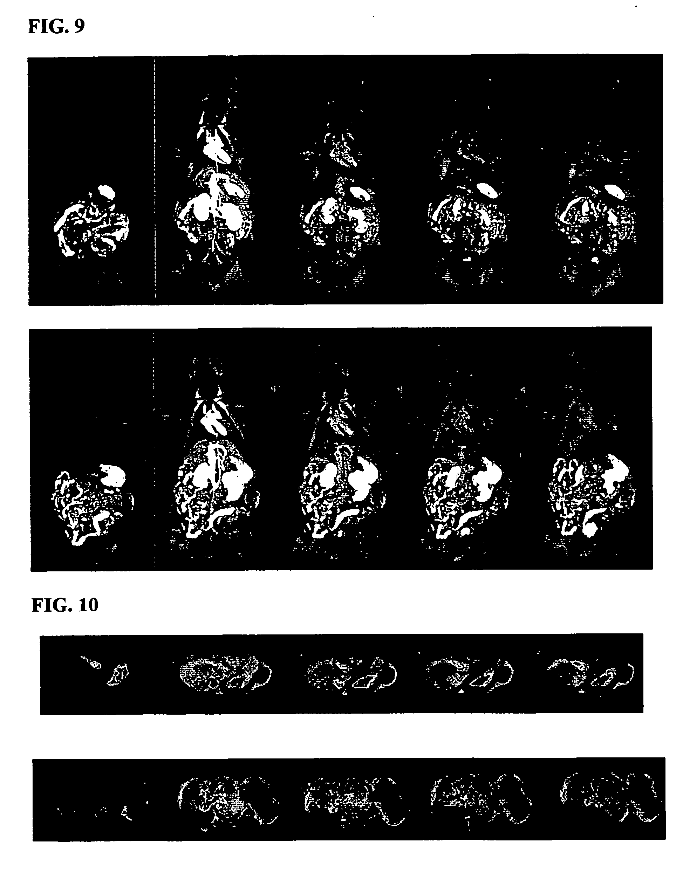

FIG. 9. 3D MIP images for animals receiving polymer conjugates with Mce6 (Pol1, upper panel) and control (Pol2, lower panel).

FIG. 10. 2D Spin echo images for Pol1 (upper panel) and Pol2 (lower panel) showing the accumulation of contrast agent in tumor tissues, post treatment.

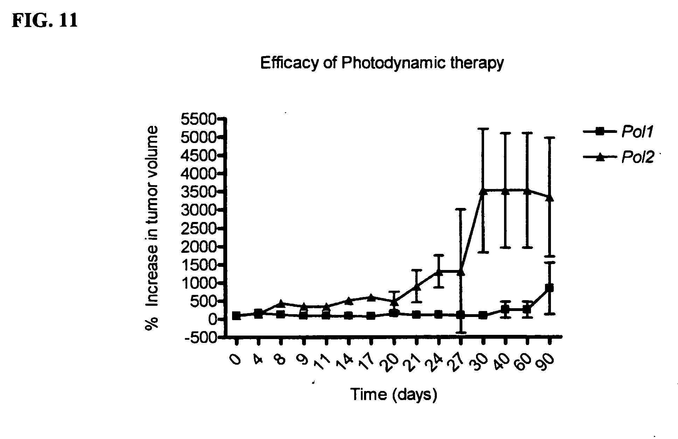

FIG. 11. An illustration of the efficacy of photodynamic therapy.

### DETAILED DESCRIPTION OF THE INVENTION

"Treatment," as used herein, is defined as including the provision of a dose of the polymer-photosensitizer conjugate in conjunction with irradiation using an energy source yielding a decrease in the number of cells in the target tissue or decrease in the size, shape, or mass of the target tissue. Treatment of a tumor or cancer cells, as used herein, is defined as including the provision of a dose of the polymer-photosensitizer conjugate in conjunction with irradiation using laser energy enabling a decrease in the size, shape, mass, viability or number of viable cancer or tumor cells.

As used herein, "effective amount" means an amount of the polymer-photosensitizer conjugate and laser energy administered to the subject that is effective to improve, prevent, or treat the disease condition in the subject.

The pharmaceutical carriers acceptable for the purposes of this invention include carriers that do not adversely affect the drug, the host, or the material comprising the drug delivery device. Suitable pharmaceutical carriers include sterile water, saline, dextrose, dextrose in water or saline condensation products of castor oil and ethylene oxide (combining about 30 to 35 moles of ethylene oxide per mole of castor oil), liquid acid, lower alkanols, oils such as corn oil, peanut oil, sesame oil, with emulsifiers such as mono- or diglyceride of a fatty acid; or a phosphatide, *e*.*g*., lecithin; glycols, polyalkylene glycols, aqueous media in the presence of a suspending agent, for example, sodium carboxymethyl cellulose, sodium alginate, poly(vinylpyrrolidone) alone, or with suitable dispensing agents such as lecithin, polyoxyethylene stearate. The carrier may also contain adjuvants such as preserving agents, stabilizing agents, wetting agents, emulsifying agents together with penetration enhancers and the polymer-photosensitizer conjugate.

In general, the various embodiments of the present invention relate to a novel polymer-photosensitizer drug conjugate for photodynamic therapy, wherein the polymer paramagnetically labeled conjugate is Gd-DOTA labeled poly-(L-glutamic acid)-Mce₆. In an embodiment, the conjugated polymer comprises PLGA-Mce₆-[(Gd-DOTA)-hexane diamine].

As discussed above, photodynamic therapy (PDT) is common in the art and in general, the polymer-photosensitizer drug conjugates of the present invention can be used in various methods of PDT, such as those disclosed in US 5,829,448, US 5,736,563, US 5,630,996, US 5,482,698, and US 6,889,723. In an embodiment, the PDT of the invention is a MRI guided therapy and the embodiment of the polymer-photosensitizer drug conjugates is contrast enhanced.

The present invention includes a delivery system as defined in claim 1; precise location of target tissue with contrast-enhanced MRI or other imaging modalities; and image-guided irradiation of target tissue with laser energy. This allows for precise location of tumor tissue and non-invasive treatment of target tissues with PDT.

Accordingly, in one embodiment, a drug delivery system of the present invention comprises a MRI contrast agent, and a photosensitizer covalently bonded to a polymer wherein the polymer is poly-L-glutamic acid, the contrast agent is Gd-DOTA, and the photosensitiser is mesochlorin e₆.

In an embodiment, said delivery system carries or conveys the contrast agent and photosensitizer into or adjacent the target site. In an embodiment, the delivery system is conveyed to a target site that is a target tissue and/or a target cell.

Further embodiments localize the target tissue by means and/or method common in the art. In an embodiment, the target site is localized by contrast-enhanced MRI or other form of MRI. Other embodiments direct the delivery system to the target site.

An energy source, such as a laser, other energy pulse, beam, and/or photo means, is then directed to/applied to/illuminates the target site. The energy pulse activates the photosensitizer, thereby killing and/or destroying the target site. In an embodiment, the energy pulse initiates or causes the photosensitizer to generate a reactive specie(s) that kills and/or immobilizes the target site.

Accordingly, various embodiments of methods of the present invention generally comprise a method for killing or destroying a target site *in vitro*, said method comprising: administering *in vitro* a pharmaceutically effective amount of a delivery system, said delivery system comprising a contrast agent and a photosensitizer as defined in claim 1; conveying the delivery system to the target tissue; localizing the target tissue with an imaging modality; and illuminating the target tissue and killing or destroying the target site. Further embodiments comprise a method for cosmetically treating a target site on a subject, said method comprising: administering to the subject a pharmaceutically effective amount of a delivery system, said delivery system comprising a contrast agent and a photosensitizer; conveying the delivery system to the target tissue; and illuminating the target tissue and treating the target site.

Delivery systems of the present invention may be administered by any method and/or applicator known in the art. In one embodiment, a delivery system of the present invention is to be administered by injection, such as by a syringe, needleless injector. In an alternate embodiment, a delivery system of the present invention is administered by application, such as pouring, wiping, smearing. In an alternate embodiment, a delivery system of the present invention is administered orally.

In an embodiment the step of administering to the subject a pharmaceutically effective amount of the delivery system of the present invention comprises administering a pharmaceutically effective amount of a magnetic resonance imaging contrast agent labeled polymer-photosensitizing conjugate. In an embodiment, the a pharmaceutically effective amount of a magnetic resonance imaging contrast agent labeled polymer-photosensitizing conjugate is poly-(*L*-glutamic acid)-(2*S*, 3*S*)-18-carboxy-20-(carboxymethyl)-8, 13-diethyl-3, 7, 12, 17-tetramethyl-chlorin-2-propionate-[(Gd-1, 4, 7,10-tetraazacyclododecane-N,N',N",N"'-tetraacetate)-hexane diamine].

The delivery systems of the present invention are used to treat or provide treatment to a desired tissue or cell, such as malignant cells, such as cancer and/or tumor cells, moles, warts, growths.

In another embodiment of the present invention, a method of synthesis of a novel MRI contrasting agent(s) conjugated to photosensitizers as defined in claim 7 useful in the treatment of target tissues in a subject is provided. In one aspect of the invention, the complexes of the present invention target rapidly dividing cells, such as tumor cells or cancerous cells. In another aspect of the invention, the complexes of the present invention may target other tissue-specific cells based on the identity of the probe linked to the photosensitizing agent. Accordingly, an embodiment of the present invention comprises the use of a pharmaceutically acceptable composition for the manufacture of a medicament for the treatment of a tumor or cancerous tissue or cancerous cells.

A method of synthesis of an exemplary conjugate comprises the steps of: A method of synthesizing poly-(*L*-glutamic acid)-(2*S*, 3*S*)-18-carboxy-20-(carboxymethyl)-8, 13-diethyl-3, 7, 12, 17-tetramethyl- chlorin-2-propionate-[(Gd-1, 4, 7,10-tetraazacyclododecane-N,N',N",N"'-tetraacetate)-hexane diamine], comprising:
reacting γ-benzyl glutamate with triphosgene to form the *N*-carboxy anhydride of
γ-benzyl glutamate;
reacting the *N*-carboxy anhydride of γ-benzyl glutamate with tributylamine to form poly-(benzyl glutamate);
reacting poly-(benzyl glutamate) with hydrogen bromide to form poly-(*L*-glutamate);
reacting poly-(*L*-glutamate) with *N*-hydroxysuccinimide to form poly-(*L*-glutamic acid)-*N*-hydroxy succinimide;
reacting poly-(*L*-glutamic acid)-*N*-hydroxy succinimide; with (2*S*, 3*S*)-18-carboxy-20-(carboxymethyl)-8, 13-diethyl-3, 7, 12, 17-tetramethyl- chlorin-2-propionate and 1, 4, 7,10-tetraazacyclododecane-N,N',N",N"'-tetraacetate-hexane diamine; and
reacting the poly-(*L*-glutamic acid)-(2*S*, 3*S*)-18-carboxy-20-(carboxymethyl)-8, 13-diethyl-3, 7, 12, 17-tetramethyl- chlorin-2-propionate-[(Gd-1, 4, 7,10-tetraazacyclododecane-N,N',N",N"'-tetraacetate)-hexane diamine] with gadolinium acetate.

One of skill in the art will understand that the composition of the present invention will not be limited to targeting of only cancer cells or tumor tissue, but rather any malignancy

The high molecular weight poly-glutamic acid polymers show higher relaxivity and higher accumulation in tumor tissue after 24 hours as compared to two hours which is indicated by an increase in contrast enhancement. The amount of polymer in or about the tumor can aid in determining the exact tumor volume to be irradiated, such as with the use of an imaging modality. Accordingly, the time of treatment may vary according to the size of the malignancy, the strength of the illumination, energy beam, and/or pulse, the depth of the malignancy in the tissue, the composition of the malignancy in the tissue, and/or the like. In an embodiment, the malignancy is treated with laser at 650 nm for about 1 to about 25 minutes. However, in general, any size and/or strength laser may be used and/or modified to be used with various embodiments provided the laser does not cause excessive damage to surrounding tissues when used within correct parameters, such as, beam width and/or time.

In various embodiments, illumination of the target site is delayed until time has been given for the administered delivery system to localize the target site. In an embodiment, a delay from between 0.1 hours to 36 hours occurs. In an alternate embodiment, a delay from about 1 hour to 24 hour occurs. In an alternate embodiment, a delay of about 18 hours occurs.

In an embodiment, a polymer-photosensitizer conjugate of the present invention is administered in multiple doses. In various other embodiments, a target site can be illuminated multiple times or through multiple procedures.

In various embodiments, images of the target site are taken. In an embodiment, an image is taken prior to administration of a delivery vehicle. In an alternate embodiment, an image is taken upon administration of a delivery vehicle. In alternate embodiment, multiple images are taken at varying time points. In various embodiments, an image is used to provide data, to provide direction, to monitor progress.

In the present invention, the polymer used is poly-(*L*-glutamic acid) with DOTA-Gd as the contrast agent and Mce₆ as the photosensitizer drug.

The present invention also discloses a method of preparing a pharmaceutical composition useful in, among other things, treating cancer. In an embodiment, the composition comprises a PLGA-Mce₆-[(Gd-DOTA)]. In an embodiment, the composition comprises the compound PLGA-Mce₆-[-1,6-hexanediamine-(Gd-DOTA)]. Various PLGA-Mce₆-(Gd-DOTA) conjugates contain a disulfide linker between the contrast agent and the polymeric carrier, allowing rapid clearing of the remaining Gd complex after treatment from the subject. (See reference 17, Lu, *et al*. for formation of such disulfide linkages which provide biodegradability characteristics of the conjugates.) Other biodegradable linkers similar to this are known in the art and it is understood that one of skill in the art is able to substitute other such linkers to provide a biodegradable character to the conjugate. Such biodegradable polymeric -conjugates are covered by the present invention and constitute a further exemplary embodiment of the present invention.

Accordingly, various embodiments of the present invention comprise a pharmaceutical composition useful in the treatment of a target tissue in a subject, said pharmaceutical composition comprising: a pharmaceutically acceptable amount of the compound poly-(*L*-glutamic acid)-(2*S*, 3*S*)-18-carboxy-20-(carboxymethyl)-8, 13-diethyl-3, 7, 12, 17-tetramethyl- chlorin-2-propionate-[(Gd-1, 4, 7,10-tetraazacyclododecane-N,N',N",N"'-tetraacetate)-hexane diamine].

Further embodiments comprise the use of a pharmaceutically acceptable composition comprising poly-(*L*-glutamic acid)-(2*S*, 3*S*)-18-carboxy-20-(carboxymethyl)-8, 13-diethyl-3, 7, 12, 17-tetramethyl- chlorin-2-propionate-[1,6-hexanediamine-(Gd-1, 4, 7,10-tetraazacyclododecane-N,N',N",N"'-tetraacetate)] in the manufacture of a medicament for the treatment of a target site.

Further embodiments comprise the use of a pharmaceutically effective amount of a magnetic resonance imaging contrast agent labeled polymer-photosensitizing conjugate of the present invention to localize or locate a target site. In one embodiment, the agent is a poly-(*L*-glutamic acid)-(2*S*, 3*S*)-18-carboxy-20-(carboxymethyl)-8, 13-diethyl-3, 7, 12, 17-tetramethyl- chlorin-2-propionate-[1,6-hexane-diamine-(Gd-1, 4, 7,10-tetraazacyclododecane-N,N',N",N"'-tetraacetate)].

Further embodiments of the present invention comprise various systems. In one embodiment, a system for localizing a target tissue is disclosed, the system comprising: a Gd-DOTA, mesochlorin e6, and an imaging modality. In an alternate embodiment, a system for treating a target site is disclosed, the system comprising: a Gd-DOTA, mesochlorin e6, an imaging modality; and a laser. Further embodiments of systems of the present invention may comprise an applicator for applying the delivery vehicle.

To further illustrate the invention, the following illustrative examples are provided.

### EXAMPLES

### Materials and Methods

γ-benzyl-glutamate was purchased for VWR (West Chester, PA). Extra dry solvents; Tetrahydrofuran (THF), Ethyl acetate, and Methylene chloride were purchased from Arcos Organics, NJ. Meso chlorin e₆ and Cyclen were purchased from Macrocyclic Inc. Gd (OAc)₃ and di-*tert*-butyl dicarbonate (*t*-boc), tert-butyl bromoacetate, N-ethyl diisopropylamine, and mono-boc-1,6-diamino hexane were purchased from Alfa Aesar (Ward Hill, MA). [1-ethyl-3-(3-Dimethylaminopropyl)carbodiimide HCl] (EDC) was purchased from TCI America (Portland, OR). Silica gel, mesh size 230-400 was purchased from Natland International Corp., NC. All solvents were purchased from Fisher Scientific and used without further purification unless otherwise stated. Spectra/Por regenerated cellulose membranes (MWCO=16-18kDa) were purchased from Spectrum Laboratories (Rancho Dominguez, CA). PD-10 desalting columns were purchased from Amersham Bioscience (Uppsala, Sweden).

Cell line (MDA-MB-231), LH-15 media containing 2µM glutamine and Trypsin were purchased from American Type Culture Collection (ATCC, Manassas VA). Female nu/nu athymic mice were purchased from NCI (Frederick, MD). Animals were maintained according to IACUC, University of Utah guidelines.

Molecular weight determination was carried out by Size Exclusion Chromatography (SEC) on an AKTA FPLC system with a Superdex 200/ Superose 6 column equipped with UV and refractive index detectors (Amersham Biosciences Corp., Piscataway, NJ). The FPLC columns were calibrated with poly[N-(2-hydroxypropyl)methacrylamide] standards. ¹H NMR spectra were acquired on a Varian INOVA 400 at 400MHz at 25°C. ESI-MS spectra were acquired on a PE Sciex API III Mass Spectrometer at the University of Utah Mass Spectrometry and Proteonomic Core Facility. Mesochlorin content was determined on Cary Win UV-Vis spectrometer. Gd content was determined using Inductively Coupled argon Plasma-Optical Emission Spectrometer (ICP-OES) (Perkin Elmer, Norwalk, CT, Optima 3100XL). T1 relaxivity measurements for the final conjugates were acquired on a Siemens Trio 3T MRI scanner using standard inversion recovery sequence. Photodynamic therapy was carried out via Diode Odyssey laser (650nm) (CAO Group, UT)

### A. Synthesis of paramagnetically labeled drug- polymer complexes

### 1. Synthesis of poly-(L-glutamic acid) active ester (PLGA-OSu).

**a. Synthesis of poly-L-glutamic acid (PLGA).** High molecular weight poly-(L-glutamic acid) was synthesized by methods previously described. Briefly, the first step in the synthesis involved the formation of N-carboxy anhydride by the reaction of y- benzyl glutamate (15g, 0.06mol) with triphosgene (9.37g, 0.03mol) in THF (15ml). The reaction was carried out at 50°C for 3 hours under N₂ and the reaction mixture was poured into n-hexane (150ml) to precipitate the NCA. The product was recrystallized by dissolving in ethyl acetate (10ml) and reprecipitating in n-hexane. NCA of γ- benzyl glutamate was obtained in high yield and purity. M.P= 110°C

For the synthesis of high molecular weight poly-(benzyl- γ-glutamate) [PBLG], ring opening polymerization was carried out by reaction of NCA (3G, 11.4mol) in 18ml ethyl acetate: dichloromethane mixture (1:6 v/v), using tributylamine 10% solution in dichloromethane) as the initiator (M/I= 100/1). The reaction was maintained at 30C for 3 hours and at R.T for 24 hours. PBLG was precipitated in methanol: ether (2:1) mixture, filtered, washed with ether and dried in vacuum.

The final step involved removal of the benzyl group on PBLG, dissolved in dichloromethane (1.0gm in 300ml), by bubbling HBr through the solution until poly-L-glutamic acid precipitated out. HBr gas was produced by the reaction of Tetralin and Bromine. PLGA was further purified using acetone. Molecular weight determination was carried out using SEC on Superdex 200 column equipped with UV and refractive index (RI) detectors. Absence of UV peak, corresponding to RI peak confirmed near complete deprotection of PLGA.

**b. Synthesis of PLGA-OSu.** The poly-(L-glutamic acid) was reacted with N-hydroxy succinimide (NHS) to form the succinic acid ester. Briefly, PLGA and NHS were dissolved in DMF and a coupling agent, EDC was added to initiate the reaction. The active ester was precipitated in acetone and dried under vacuum. Presence of ester was confirmed by NMR.

### 2. Synthesis of Gd-DOTA- 1,6- diamino hexane (linker).

**a. Synthesis of DO₃A.** DO₃A was synthesized via cyclen in 2 steps according to methods previously described but with few changes to obtain DO₃A of high purity.

In the first step, cyclen (1.72gm, 10mmol) and DIPEA (10eq, 100mmol, 12.9gm) were dissolved in 80ml chloroform. On complete dissolution, the reaction mixture was reacted with tert-butyl bromoacetate in chloroform in two steps. For first addition *tert*-butyl bromoacetate (2eq, 20mmol, 3.92gm) dissolved in 40ml chloroform was reacted by dropwise addition to the solution with continuous stirring. On complete addition, the reaction was stirred for 12 hours. For second addition, *tert*-butyl bromoacetate (1eq, 10mmol, 1.96gm) dissolved in 20ml was added dropwise to the reaction mixture. The reaction was stirred for another 18 hours at R.T. The solution was concentrated in vacuum, solid dissolved in minimum amount of Methylene chloride and applied to Silica gel column for further purification. Gradient elution using ethyl acetate: methanol (100%, 50:1, 20:1 and 10:1) as eluent system was carried out. TB-cyclen (*tert*-butyl protected cyclen) was obtained in 20:1 fraction.

TB cyclen was deprotected using minimum amount of cold trifluoroacetic acid (25°C, overnight) to form DO₃A. The product was purified using diethyl ether.

**b. Synthesis of N-boc-1,6-diaminohexane bromoacetate (Intermediate).** In a one step process, N-boc-1,6-diaminohexane (1gm, 4mmol) was reacted at 0°C with N-hydroxy succinimide ester of bromoacetic acid (1.32gm, 5mmol) in dichloromethane (30ml). After complete addition, the reaction was stirred for 24 hours at 25C. The bromoacetate ester purified by washing with NaOH (2x 50ml) and water (2x 50ml), extraction in organic solvent and concentrated to dryness.

**c. Synthesis DOTA-1,6-diaminohexane.** The linker was synthesized by the reaction of DO₃A (0.7gm, 2mmol) with the N-boc-1,6-diaminohexane bromoacetate (1.36gm, 4mmol) in 20ml of methanol: triethylamine solvent mixture (2:1) and excess K₂CO₃ (2.76gm, 20mmol). The reaction was carried out for 24 hours at room temperature. The solvent was evaporated and the product dissolved in water, acidified with dilute HCl. The pH was adjusted between 2-3 to convert all the acid into salt and dissolve inorganic salts like KBr. The aqueous solution was evaporated in vacuum and the linker was extracted as a salt from methanol: triethylamine (2:1) mixture. **3. Synthesis of Gd(III)-DOTA-linker-PLGA and Gd-DOTA-linker-PLGA-Mce₆ complexes.**

**a. Synthesis of DOTA-linker-PLGA and DOTA-linker-PLGA-Mce₆** conjugates. PLGA active ester (PLGA-NHS, 200mg, 542mmol) was dissolved in DMF (3ml) with mesochlorin e₆ (24mg, 37mmol) and reacted for 30 minutes. DOTA-linker (300mg, 600mmol) was then added to the solution. Excess DMAP was incorporated after each addition to initiate reaction. The reaction was carried out for 24 hours at room temperature. At the end of reaction, DMF was evaporated under vacuum, the residue was dissolved in water. DOTA-linker-PLGA conjugates, for use as controls were synthesized in a similar manner, without addition of any Mce₆. Complexation of the polymer conjugates with Gd (III) was carried out without further purification.

**b. Synthesis of Gd (III)-DOTA-linker-PLGA-Mce₆ (*Pol₁*)and Gd (III)-DOTA-linker-PLGA (*Pol₂*).** Aqueous solution of polymer conjugates in DI water was reacted with excess Gd (III) acetate at pH 8. After it was stirred overnight, the excess Gd was precipitated out as Gd₂O₃ by increasing the pH of the solution 11 with dilute NaOH. The oxide was precipitated out and the pH of the solution was adjusted between 5.5- 6. The final solution was concentrated under vacuum and applied to a column. The eluate was lyophilized.

### B. Characterization of paramagnetically labeled polymer-photosensitizer conjugate (table 1)

**Determination of molecular weight.** Molecular weights of the polymeric complexes were determined using size exclusion chromatography on AKTA FPLC system (Amersham Inc). The complexes were dissolved in TRIS buffer pH 7.4 and filtered through 0.2µ membrane before applying to the column.

Determination **of relaxivity.** T1 relaxivity of the polymer complexes, *Pol1* and *Pol2*, was determined on a Siemens Trio 3T scanner using a standard inversion recovery sequence with varying TI of 22- 1600 ms.

**Determination of Gd content.** Gd (III) of *Pol₁* and *Pol₂* content was determined by Inductively Coupled Plasma- Optical Emission Spectrometry (ICP-OES) using Gd standards to construct standard curve.

**Determination of Mce₆ content.** Mesochlorin e₆ content for *Pol₁* was determined by UV spectrophotometry. Mce₆ standard the polymer conjugate solutions were prepared in methanol and UV absorbance measured at 650nm.

### C. Cell cultures.

MDA-MB-231 (ATCC) human breast carcinoma cell line was grown in LH-15 culture media with 2mM glutamine and 10% FBS, in 5% CO₂. The cells were harvested at confluence and counted after staining with trypan blue. 2x10⁶ cells were injected per animal to generate tumor models.

### D. Animal models.

Female athymic nu/nu mice were purchased from NCI (Frederick, MD) at the age of 6 weeks. To generate tumor models, each mouse was injected subcutaneously with 2x10⁶ MB-231 cells in 100µl of culture media mixed with 100µl of BD MATRIGEL, a solubulized basement membrane preparation extracted from EHS mouse sarcoma, in the flank region. Magnetic resonance imaging and photodynamic therapy studies were carried out at an average tumor size of 25- 40mm³.

**Table 1 (data is for mice used in the results illustrated in Figure 6) Determination of Gd (ICP) and Meso Chlorin (UV) Content**

| | **Mmol/G** | **Mol%** |
|---|---|---|
| **Gd** | 0.62 | 20 |
| **Mce6** | 0.078 | 2.5 |

### Example I

### Animal Tumor Model Tests

To generate xenografts, six female athymic nu/nu mice were injected with MDA-MB231 human breast carcinoma cells (ATCC). About 1 to 3x10⁶ cells were mixed with MATRIGEL (BD Biosciences) and were injected per mouse. The mice were observed for tumor growth. Tumor volume was measured using digital calipers. The mice were selected for imaging and therapy when the tumor volume was approximately 20 mm³.

### Example II

### Magnetic Resonance Imaging of Animal Models and Administration of Conjugates

MR imaging was performed on a Siemens Trio 3T MRI scanner. The animals were anesthetized by intra-peritoneal or intra-muscular injection of the anesthetic (mixture of ketamine - 80 mg/kg body weight and xylazine - 12 mg/kg body weight).

For imaging purposes, the animals were placed in the human wrist coil and pre-contrast images (prior to the injection of the polymer chelate system containing the drug and the contrast agent) were obtained using a 3D FLASH (Fast Low Angle Shot) sequence.

After obtaining the pre-contrast images, the macromolecular complexes were injected at a dose of 0.09 mmol Gd/kg body weight and 6 mg/kg of Mce₆ by tail vein injection. Image acquisition was set at 5, 30, 60, 120 minutes and 24 hours post injection. Animal imaging was performed using the multi-phase T1 weighted sequence. The repetition time was about 6 milliseconds to 2 milliseconds, respectively; consistent with the 4 to 8 cm FIV (field of view) and 0.5 mm slice thickness chosen for each animal. The imaging volume was acquired before injection and in rapid succession after injection. Each acquisition required about 30 seconds.

Similar dosage amounts are expected to be effective in other subjects, such as humans. In humans, dosage amounts may include, for instance, 0.01 to 0.03 mmol Gd/kg body weight. Similarly, dosage amounts are expected to be effective in other subjects, such as humans wherein the dosage amount may include, for example, 1 to 10 mg/kg body weight of Mce₆. However, the dosage may be adjusted as determined by routine experimentation.

### Example III

### Specific Illumination of the Target Tissue in Animal Model

Once the contrast enhancement is observed, the tumor is irradiated using laser at a wavelength of 650 nm corresponding to the excitation wavelength of Mce₆.

The position and the accumulation of the macromolecular complexes in the tumor were observed with MRI. Then the tumor was irradiated, 2 hours and 18 hours post-injection, using light of wavelength 650 nm (Denlaser) with an input of 220 mW/cm² energy and 0.5 mm aperture for irradiation. The time of irradiation was 15 minutes. The mice were then kept away from direct light to avoid extraneous irradiation and the reduction in tumor volume was measured.

Results are depicted in the magnetic resonance images of FIG. 6, showing images of tumors in athymic nu/nu mice at pre-, 5, 30, 60, 120 minutes and 24 hours post-injection. The slices are through the heart and tumor separately, for mouse 1, and through the heart and tumor simultaneously, for mouse 2. In both mice, the 24 hour images show contrast enhancement through the tumor, compared to pre-contrast, while there is contrast enhancement also in the heart at five minutes post-injection. In total, 6 mice were examined using the above methodology, all yielding similar results. These results indicate that the conjugates are capable of localizing in the target tissues within 24 hours.

### Example IV.

**Synthesis and Physicochemical characterization of polymer conjugates and controls for Example VII.** Poly-(-glutamic acid) conjugates containing either Mce₆ and Gd(III) DOTA (*Pol₁* and *Pol₃*) or only Gd(III) DOTA (*Pol₂*) were synthesized from PLGA of the same molecular weight, Mw = 82kDa. Different ratios of drug and contrast agent were evaluated (not shown) to produce final conjugates with optimum content for both active moieties. Mesochlorin e₆ was used due to its hydrophobic nature and potential to be excited at higher wavelengths of light, leading to better penetration of the light. Gd-DOTA, a clinically approved contrast agent, was chosen due to higher stability as compared to acyclic metal chelates. Physicochemical characterization, including molecular weights, PDI, mesochlorin e₆ and Gd content, and relaxivity for the polymeric conjugates and controls is given in Table 2 below. The polymer conjugates with and without the drug showed higher relaxivity as compared to low molecular weight counterparts.

**Table 2. (results are for mice in Figures 7-10)**

| Polymers | | | | | | |
|---|---|---|---|---|---|---|
| | Mn | Mw | PDI | Gd | Mce6 | Relaxivity |
| | (kDa) | (kDa) | | (mmol/G) | (mmol/G) | (mM-1sec-1) |
| | | | | | | |
| PLGA | 67 | 82 | 1.2 | | | |
| Pol1 (Gd-DOTA-PG - Mce6) | 34 | 49 | 1.4 | 0.62 | 0.078 | 8.46 |
| Pol2 (Gd-DOTA-PG) | 49 | 101 | 2.06 | 0.92 | - | 8.33 |

**MR imaging.** Contrast enhancement was observed in two groups of 6 mice with *Pol₁* and *Pol₂.* Figure 7 and Figure 8 show 2D coronal MR images through tumor and heart respectively. The upper panel of Figure 7 represents mice with *Pol₁* and the lower panel upper represents mice with *Pol₂.* The upper panel of Figure 8 represents mice with *Pol₁* and the lower panel represents mice with *Pol₂.* As compared to pre-contrast, there was an increased contrast in all the tissues, including tumor, heart and liver (data not shown) at 5 minutes post injection for both *Pol₁* and *Pol₂.* There was higher contrast in heart at all times for the controls while similar enhancement was observed for both in tumor tissues at all times. The higher signal in heart may be due to high molecular weight fractions of the control polymer.

Semi-quantitative evaluation of signal intensity in the tumor tissue for both the polymers indicated an increased contrast by 2 hours post injection. Increase in signal intensity of tumor for both groups as compared to precontrast at 2 hours is 100%. Signal intensity at 18 hours post injection was at least 30% higher than precontrast for both *Pol₁* and *Pol₂.* Quantitative evaluation of signal intensities using IDL was performed to corroborate the data obtained from semi-quantitative analysis.

**Efficiency of Photodynamic therapy.** Reduction in tumor volume for mice belonging to the first group, 6 mice received polymer- drug- CA (drug) and 6 mice received polymer- CA conjugates (control).

Figure 11 illustrates that a reduction in tumor volume or slowed tumor growth was observed for mice receiving *Pol₁* while the mice injected with *Pol₂* (control group) showed rapid increase in tumors. 4 out of 6 the mice in the drug group survived for the time period of the experiment (90 days). Out of the 6 mice in the control group, 5 had tumors larger than 10% of the body weight and were sacrificed.

**MR imaging post treatment.** For post treatment MR imaging, a high molecular weight contrast agent, (Gd-DTPA)-cystine copolymer (GDCP) was used to determine the difference in tumor uptake for treated and untreated mice. This agent has been shown in previous work to produce relatively strong contrast enhancement in angiogenic blood vessels in similar tumor models. The molecular weight and relaxivity of the polymer were similar to that of *Pol₁,* 34kDa and 5.33 mM⁻¹sec⁻¹.

Figure 9 illustrates 3D MIP echo MR images of mice receiving *GDCC* at 2, 5, 10, 15, 30 minutes post injection. Figure 10 illustrates 2D Spin echo MR images of mice receiving *GDCC* at 2, 5, 10, 15, 30 minutes post injection. The tumors showed a rim enhancement for mice in both groups. The mice in control group however showed higher contrast enhancement, indicative of absence of treatment.

**Results.** The decrease in tumor volumes in mice receiving drugs was significantly different from the controls. Further, the above results illustrate that the time of irradiation can be determined and adjusted using the dynamic data obtained from, for example, MRI, leading to an increased efficiency for enhanced site-directed photodynamic therapy.

### REFERENCES

(1) Caravan P., Ellison J. J., McMurry T. J., and Lauffer R. B. (1999) Gadolinium (III) chelates as MRI contrast agents: Structure, dynamics, and applications. Chem. Rev. 99, 2293-2352.
(2) Schuhmann-Giampieri G., Schmitt-Willich H., Frenzel T., Press W. R. and Weinmann H. J. (1991) In vivo and in vitro evaluation of Gd-DTPA-polylysine as a macromolecular contrast agent for magnetic resonance imaging. Invest. Radiol. 26, 969-974.
(3) Weissleder R., Bogdanov A. Jr., Tung C. H., Weinmann H. J. (2001) Size optimization of synthetic graft copolymers for in vivo angiogenesis imaging. Bioconjugate Chem. 12, 213-219.
(4) Rongved P. and Klaveness J. (1991) Water-soluble polysaccharides as carriers of paramagnetic contrast agents for magnetic resonance imaging: synthesis and relaxation properties. Carbohydr. Res. 214, 315-323.
(5) Gibby W. A., Bogdan A., and Ovitt T. W. (1989) Cross-linked DTPA polysaccharides for magnetic resonance imaging. Invest. Radiol. 24, 302-309.
(6) Wiener E. C., Brechbiel M. W., Brothers H., Magin R. L., Gansow O. A., Tomalia D. A. and Lauterbur P. C. (1994) Dendrimer-based metal chelates: a new class of magnetic resonance imaging contrast agents. Magn. Reson. Med. 31, 1-8.
(7) Bryant L. H., Brechbiel M. W., Wu C., Bulte J. W. M., Herynek V., and Frank J. A. (1999) Synthesis and relaxometry of high generation (G = 5, 7, 9 and 10) PAMAM dendrimer-DOTA-gadolinium chelates. J. Magn. Reson. Imaging 9, 348-352.
(8) Kobayashi H., Sato N., Kawamoto S., Saga T., Hiraga A., Haque T. L., Ishimori T., Konishi J., Togashi K., and Brechbiel M. W. (2001) Comparison of the macromolecular MR contrast agents with ethylenediamine-core versus ammonia-core generation-6 polyamidoamine dendrimer. Bioconjugate Chem. 12, 100-107.
(9) Lauffer R. and Brady T. J. (1985) Preparation and water relaxation properties of proteins labeled with paramagnetic metal chelates. Magn. Reson. Imaging 3, 11-16.
(10) Roberts H. C., Saeed M., Roberts T. P., Muhler A., Shames D. M., Mann J. S., Stiskal M., Demsar F., and Brasch R. C. (1997) Comparison of albumin-(Gd-DTPA)30 and Gd-DTPA-24-cascade-polymer for measurements of normal and microvascular permeability. J. Magn. Reson. Imaging 7, 331-338.
(11) Schuhmann-Giampieri G., Schmitt-Willich H., Frenzel T., Press W. R. and Weinmann H. J. (1991) In vivo and in vitro evaluation of Gd-DTPA-polylysine as a macromolecular contrast agent for magnetic resonance imaging. Invest. Radiol. 26, 969-974.
(12) Bogbanov A. A. Jr., Weissleder R., Frank H. W., Bogdanova A. V., Nossif N., Schaffer B. K., Tsai E., Papisov M. I. and Brady T. J. (1993) A new macromolecule as a contrast agent for MR angiography: preparation, properties, and animal studies. Radiology 187, 701-706.
(13) Franano F. N., Edwards W. B., Welch M. J., Brechbiel M. W., Gansow O. A. and Duncan J. R. (1995) Biodistribution and metabolism of targeted and nontargeted protein-chelate-gadolinium complexes: evidence for gadolinium dissociation in vitro and in vivo. Magn. Reson. Imaging 13, 201-214.
(14) Rebizak R., Schaefer M. and Dellacherie E. (1998) Polymeric conjugates of Gd 3+ -diethylenetriaminepentaacetic acid and dextran 2. Influence of spacer arm length and conjugate molecular mass on the paramagnetic properties and some biological parameters. Bioconjugate Chem. 9, 94-99.
(15) Kobayashi H., Kawamoto S., Jo S.-K., Bryant H. L. Jr., Brechbiel M. W. and Star R. A. Macromolecular MRI contrast agents with small dendrimers: pharmacokinetic differences between sizes and cores. Bioconjugate Chem., in press.
(16) Kobayashi H., Sato N., Kawamoto S., Saga T., Hiraga A., Ishimori T., Konishi J., Togashi K. and Brechbiel M. W. (2001) Novel intravascular macromolecular MRI contrast agent with generation-4 polyamidoamine dendrimer core: accelerated renal excretion with co-injection of lysine. Magn. Reson. Med. 46, 457-464.
(17) Lu Z.-R., Wang, X., Parker D. L., Goodrich K. C. and Buswell H. R. (2003) Poly(L-glutamic acid) Gd(III)-DOTA Conjugate with a Degradable Spacer for Magnetic Resonance Imaging. Bioconjugate Chem. 14, 715-719.
(18) Parker, D. L., Christian, B. A., Goodrich, K. C., Alexander, A. L., Buswell, H. R. and Yoon, C. Improved accuracy in T1 measurements. Proc. ISMRM; Sydney, Australia, April, 1998; p 2171.

## Claims

1. An *in vitro* method for killing or destroying a target site comprising:
administering *in vitro* a pharmaceutically effective amount of a delivery system, wherein the delivery system comprises a contrast agent and a photosensitizer which are covalently bonded to a polymer; wherein the polymer is poly-L-glutamic acid, the contrast agent is Gd-DOTA, and the photosensitizer is mesochlorin e₆;
conveying the delivery system to the target tissue;
localizing the target tissue with an imaging modality; and
illuminating the target tissue and killing or destroying the target site.

2. The method of claim 1, wherein the delivery system is poly-L-glutamic acid-mesochlorin e₆-[1,6-hexanediamine-(Gd-DOTA)].

3. The method of claim 1, wherein the delivery system is poly-(L-glutamic acid)-(2S, 3S)-18-carboxy-20-(carboxymethyl)-8, 13-diethyl-3, 7, 12, 17-tetramethyl-chlorin-2-propionate-[1,6-hexanediamine-(Gd-1,4,7,10-tetraazacyclododecane-N,N',N",N"'tetraacetate)].

4. The method in any of claims 1, wherein the delivery system further comprises a biodegradable linker.

5. The method of claim 4, wherein the biodegradable linker is a disulfide linkage.

6. The method of anyone of claims 1-5, wherein the step of localizing the target tissue comprises using magnetic resonance imaging (MRI).

7. A composition comprising a conjugate of a contrast agent and a photosensitizer which are covalently bonded to a polymer; wherein the polymer is poly-L-glutamic acid, the contrast agent is Gd-DOTA, and the photosensitizer is mesochlorin e₆.

8. The composition of claim 7, comprising poly-L-glutamic acid-mesochlorin e₆-[1,6-hexanediamine-(Gd-DOTA)].

9. The composition of claim 7, comprising poly-(L-glutamic acid)-(2S, 3S)-18-carboxy-20-(carboxymethyl)-8, 13-diethyl-3, 7, 12, 17-tetramethyl-chlorin-2-propionate-[1, 6-hexanediamine-(Gd-1, 4, 7, 10-tetraazacyclododecane-N,N',N",N"'tetraacetate)].

10. The composition in claim 7, wherein the conjugate further comprises a biodegradable linker.

11. The composition of claim 10, wherein the biodegradable linker is a disulfide linkage.

12. A kit for localizing a target tissue, the kit comprising (1) a conjugate as claimed in claims 7-11, and (2) an applicator.

13. A kit for treating a target site, the kit comprising (1) a conjugate as claimed in claims 7-11, (2) an applicator, and (3) a laser.

14. A composition of claims 7-11 for use in the treatment of a target site.

15. A composition of claims 7-11 for use in the localisation or location of a target site using magnetic resonance imaging.

## Patentansprüche

1. In-Vitro-Verfahren zum Abtöten oder Zerstören eines Zielorts, das beinhaltet:
In-Vitro-Verabreichen einer pharmazeutisch wirksamen Menge eines Abgabesystems, wobei das Abgabesystem ein Kontrastmittel und ein Photosensibilisierungsmittel aufweist, welche kovalent mit einem Polymer gebunden sind, wobei das Polymer Poly-L-Glutaminsäure, das Kontrastmittel Gd-DOTA und das Fotosensibilisierungsmittel Mesochlorin e₆ darstellt;
Überführen des Abgabesystems an das Zielgewebe;
Lokalisieren des Zielgewebes mit einer Bildgebungsmodalität; und
Beleuchten des Zielgewebes und Abtöten oder Zerstören des Zielorts.

2. Verfahren nach Anspruch 1, wobei das Abgabesystem Poly-L-Glutaminsäure-Mesochlorin e₆-[1,6-Hexandiamin-(Gd-DOTA)] darstellt.

3. Verfahren nach Anspruch 1, wobei das Abgabesystem Poly-(L-Glutaminsäure)-(2S,3S)-18-carboxy-20-(carboxymethyl)-8,13-diethyl-3,7,12,17-tetramethyl-chlorin-2-propionat-[1,6-hexandiamin-(Gd-1,4,7,10-tetraazacyclodo-decan-N,N',N",N"'tetraacetat)] darstellt.

4. Verfahren nach Anspruch 1, wobei das Abgabesystem ferner einen bioabbaubaren Linker aufweist.

5. Verfahren nach Anspruch 4, wobei der bioabbaubare Linker eine Disulfidbindung darstellt.

6. Verfahren nach einem der Ansprüche 1-5, wobei der Schritt des Lokalisierens des Zielgewebes das Verwenden von Magnetresonanzbildgebung (MRI) beinhaltet.

7. Zusammensetzung, die ein Konjugat eines Kontrastmittels und eines Photosensibilisierungsmittels aufweist, welche kovalent mit einem Polymer gebunden sind; wobei das Polymer Poly-L-Glutaminsäure, das Kontrastmittel Gd-DOTA und das Photosensibilisierungsmittel Mesochlorin e₆ darstellt.

8. Zusammensetzung nach Anspruch 7, die Poly-L-Glutaminsäure-Mesochlorin e₆-[1,6-Hexandiamin-(Gd-DOTA)] aufweist.

9. Zusammensetzung nach Anspruch 7, die Poly-(L-Glutaminsäure)-(2S,3S)-18-carboxy-20-(carboxymethyl)-8,13-diethyl-3,7,12,17-tetramethyl-chlorin-2-propionat-[1,6-hexandiamin-(Gd-1,4,7,10-tetraazacyclododecan-N,N',N",N"' tetraacetat)] aufweist.

10. Zusammensetzung nach Anspruch 7, wobei das Konjugat ferner einen bioabbaubaren Linker aufweist.

11. Zusammensetzung nach Anspruch 10, wobei der bioabbaubare Linker eine Disulfidbindung ist.

12. Kit zum Lokalisieren eines Zielgewebes, wobei der Kit (1) ein Konjugat nach einem der Ansprüche 7-11 und (2) einen Applikator aufweist.

13. Kit zum Behandeln eines Zielorts, wobei der Kit (1) ein Konjugat nach einem der Ansprüche 7-11, (2) einen Applikator und (3) einen Laser aufweist.

14. Zusammensetzung nach einem der Ansprüche 7-11 zur Verwendung bei der Behandlung eines Zielorts.

15. Zusammensetzung nach einem der Ansprüche 7-11 zur Verwendung bei der Lokalisierung oder Verortung eines Zielorts unter Verwendung von Magnetbildgebung.

## Revendications

1. Procédé in vitro pour éliminer ou détruire un site cible, comportant les étapes consistant à :
administrer in vitro une quantité pharmaceutiquement efficace d'un système d'administration, le système d'administration comportant un produit de contraste et un photosensibilisateur qui sont liés par covalence à un polymère, dans lequel le polymère est le poly(acide L-glutamique), le produit de contraste est Gd-DOTA, et le photosensibilisateur est la mésochlorine e₆ ;
transporter le système d'administration jusqu'au tissu cible ;
localiser le tissu cible à l'aide d'une modalité d'imagerie ; et
éclairer le tissu cible et éliminer ou détruire le site cible.

2. Procédé selon la revendication 1, dans lequel le système d'administration est poly(acide L-glutamique)-mésochlorine e₆-[1,6-hexanediamine-(Gd-DOTA)].

3. Procédé selon la revendication 1, dans lequel le système d'administration est poly(acide L-glutamique)-(2S,3S)-18-carboxy-20-(carboxyméthyl)-8,13-diéthyl-3,7,12, 17-tétraméthyl-chlorin-2-propionate-[1,6-hexanediamine-(Gd-1,4,7,10-tétraazacyclododécane-N,N',N",N"'-tétraacétate)].

4. Procédé selon la revendication 1, dans lequel le système d'administration comporte en outre un lieur biodégradable.

5. Procédé selon la revendication 4, dans lequel le lieur biodégradable est une liaison disulfure.

6. Procédé selon l'une quelconque des revendications 1 à 5, dans lequel l'étape de localisation du tissu cible comporte l'utilisation d'une imagerie par résonance magnétique (IRM).

7. Composition comportant un conjugué d'un produit de contraste et d'un photosensibilisateur qui sont liés par covalence à un polymère, dans laquelle le polymère est le poly(acide L-glutamique), le produit de contraste est Gd-DOTA, et le photosensibilisateur est la mésochlorine e₆.

8. Composition selon la revendication 7, comportant poly(acide L-glutamique)-mésochlorine e₆-[1,6-hexane-diamine-Gd-DOTA)].

9. Composition selon la revendication 7, comportant poly(acide L-glutamique)-(2S,3S)-18-carboxy-20-(carboxyméthyl)-8,13-diéthyl-3,7,12,17-tétraméthyl-chlorin-2-propionate-[1,6-hexanediamine-(Gd-1,4,7,10-tétra-azacyclododécane-N,N',N",N"'-tétraacétate)].

10. Composition selon la revendication 7, dans laquelle le conjugué comporte en outre un lieur biodégradable.

11. Composition selon la revendication 10, dans laquelle le lieur biodégradable est une liaison disulfure.

12. Kit pour localiser un tissu cible, le kit comportant (1) un conjugué tel que revendiqué dans les revendications 7 à 11, et (2) un applicateur.

13. Kit pour traiter un site cible, le kit comportant (1) un conjugué tel que revendiqué dans les revendications 7 à 11, (2) un applicateur et (3) un laser.

14. Composition selon les revendications 7 à 11 pour une utilisation dans le traitement d'un site cible.

15. Composition selon les revendications 7 à 11 pour une utilisation dans la localisation ou le positionnement d'un site cible en utilisant une imagerie par résonance magnétique.
